# EUROPEAN PATENT SPECIFICATION

(11) **EP 2 432 479 B1**
(45) Date of publication and mention of the grant of the patent: **13.11.2019**
(21) Application number: 10777992.8
(22) Date of filing: 20.05.2010
(51) Int. Cl.: A61K 9/50, A61K 31/7004, A61K 33/00, A61K 33/40, A61K 35/20, A61K 38/44, A61P 31/04

(54) **EXTENDED ACTING OXYGEN GENERATING COMPOSITION FOR TREATING MICROBIAL INFECTIONS**
SAUERSTOFFERZEUGUNGSZUSAMMENSETZUNG MIT ERWEITERTER WIRKUNG ZUR BEHANDLUNG MIKROBIELLER INFEKTIONEN
COMPOSITION GÉNÉRANT DE L'OXYGÈNE À ACTION PROLONGÉE UTILISÉE POUR TRAITER LES INFECTIONS MICROBIENNES

(30) Priority: 20.05.2009 NZ 57711909
(43) Date of publication of application: 28.03.2012
(73) Proprietor: DEC International NZ Limited, Te Rapa, Hamilton 3241 (NZ)
(72) Inventor: BRAGGER, Judith, Mary, Hamilton, 3204 (NZ); CLAYCOMB, Rodney, Wayne, Hamilton, 3210 (NZ)
(74) Representative: Tomkinson, Alexandra
(86) International application number: PCT/NZ2010/000093
(87) International publication number: WO 2010/134827

(56) References cited:
- WO-A1-2009/118379
- WO-A2-2007/142542
- US-A- 4 310 116
- US-A- 5 066 497
- SANDHOLM ET AL. ET AL.: 'Glucose Oxidase (GOD) as a Source of Hydrogen Peroxide for the Lactoperoxidase (LPO) System in Milk: Antibacterial Effect of the GOD-LPO System against Mastitis Pathogens' JOURNAL OF VETERINARY MEDICINE SERIES B vol. 35, 1988, pages 346 - 352, XP009151074

## Description

### TECHNICAL FIELD

This invention relates to a medicament and treatment or prevention method.

In particular it relates to a medicament and method for treating or preventing microbial infection or an associated condition.

### BACKGROUND ART

Microbial infections can arise in a multitude of locations on an animal's body due to a wide assortment of causes. Such infections can progress into medical conditions and these as well as the underlying infections can cause numerous health problems.

Indeed, there are many types of compositions available for the treatment of surface microbial infections, or to act as disinfectants for non-medical uses. Much of these are for topical application to a surface of skin or object to be cleaned.

Whilst these may be effective for anti-microbial treatment, internal therapeutic treatment of microbial infection remains difficult to treat, are ineffective with currently available formulations and/or lead to unwanted side effects (e.g. unwanted withholding periods of milk following usage of antibiotics to treat mastitis).

The reason behind this difficulty in internal microbial treatment is not well understood. It remains a challenge to develop effective treatment formulations that not only achieve the desired result (an anti-microbial effect), but are also safe for the animal being treated and human consumption of animal produce following such treatment. Mastitis is just one condition which can arise from microbial infection. Mastitis is currently largely treated with antibiotics, however there are major disadvantages with this treatment. Unfortunately, antibiotics require withholding periods, which can increase the time and effort required by the farmer, further increasing the costs associated with mastitis and still may not effectively target the pathogens causing the mastitis. Therefore, a non-antibiotic method of controlling/killing the bacteria leading to mastitis remains a critical requirement in the dairy industry.

Resistance of the major mastitis-causing pathogens to antibiotics is another negative aspect to reliance on antibiotic treatments.

As such, many have tried to develop a non-antibiotic treatment formulation for controlling the bacteria that cause mastitis. Many attempts have been made to mimic or supplement natural microbial defense mechanisms such as the Lactoperoxidase (Lp) system. However, for unknown reasons, such treatments have continued to be ineffective at treating mastitis and other microbial infections which are internally located in the body.

The milk of mammals has evolved to contain innate natural antimicrobial components. The most commonly known of these components are leukocytes, which exist in various forms in the milk. Proteins and enzymes such as lactoferrin, lactoperoxidase, angiogenin, lysozyme and many others can act synergistically to produce antimicrobial compounds within the milk that can directly fend off microbial infection and can help mitigate the effects of mastitis.

For example, lactoperoxidase (Lp) system provides a defense mechanism, which can produce a natural bactericidal agent. However, during mastitic infection, this system, along with the suite of other immune defenses, are often unable to cope with the microbial infection. In fact, dairy cows with mastitic infection are often unable to self-cure, despite usually having elevated levels of somatic cells and defense proteins. This leaves antibiotics as still the best state-of-the-art solution for treating mastitis.

### Background to Mastitis

Although mastitis is considered to be an inflammatory disease of the mammary gland of a mammal, the inflammation is the result of infection by any of a multitude of bacteria, mycoplasmas, yeast and fungi. The range of different organisms that can cause mastitis, their varying susceptibilities to antibiotics or other treatments and the ability of the treatments to access the organisms present the greatest challenges in the treatment and prevention of mastitis. This is especially true in dairy cows, with mastitis accounting for the number one cost of production worldwide.

Bovine mastitis may be caused by Gram negative bacteria such as Escherichia coli, Klebsiella species and Enterbacter species, or by Gram-positive bacteria such as Staphylococcus aureus, Enterococci species, and Streptococci such as Streptococcus uberus, Streptococcus agalactiae and Streptococcus dysgalactiae, and by Mycoplasma bovis.

Mastitis, particularly bovine mastitis, is of considerable economic significance to the dairy industry. This is particularly due to the following:
- the high cost of the treatment,
- the loss of milk during the infected period, and subsequent withholding period following the use of antibiotics. If antibiotics are found in the milk supplied to a dairy company, the whole batch may need to be discarded, and the farmer may face large penalties.
- cross-contamination within the herd or between individual teat quarters.
- long-term loss of milk over the life of the animal due to decreased mammary capacity

To-date no one has developed an effective non-antibiotic, natural treatment method for mastitis. There have been attempts to utilise the naturally-occurring milk-derived defense proteins as antimicrobial mastitis treatments. A number of these compositions have been published as discussed below.

US5700465 discloses a method of treating mastitis comprising administering monomeric IgG2 to stimulate endogenous neutrophil function.

US20060093594 discloses a formulation based on a novel so-called "treatment for contaminant reduction" (TCR) Lf that includes a surfactant, an antioxidant, a polyphenol and at least one anionic compound.

US5834424 discloses topical administration of Lf for inhibiting gram positive bacteria.

US20060142183 discloses a composition for inhibiting β-lactamase using Lf and/or lactoferricin with a carrier.

US5466449 discloses a method of treating bacterial infection comprising administration of treatment including a dimer of lysozyme and a carrier.

US20040235711 discloses a composition for smooth transition of a lactating bovine mammary gland from lactation to dry periods containing Lf.

Combining naturally-derived milk proteins with antibiotics has also been disclosed in US5198419, wherein Lf is used to enhance betalactam antibiotics. US6562820 discloses a method of treating mastitis caused by E coli, using a dry period administration of oxazolidinone with or without a complementary dose of Lf to enhance the oxazolidinone. There have also been attempts to utilise more than one of the naturally occuring milk defense proteins in a mastitis treatment formulation.

US20070116698 discloses a composition including one of a hypohalite, hypothiocyanite or combination; at least one of Lf, Lf peptide, lysozyme, Ig or combination; and at least one growth factor.

WO9606532 discloses a bacteriostatic composition consisting of a basic milk protein or peptide in combination with a cell-wall degrading enzyme or oxidoreductase.

NZ547859 refers to the use of a combined suite of milk-derived proteins with isoelectric points greater than 6.8 as a potential treatment for mastitis. It also discloses a formulation based on these proteins that has an added cell-lysing substance or Lp substrates.

US4320116 discloses a medicament for targeting severe gastrointestinal infections including a coated percarbonate granule. The percarbonate forms hydrogen peroxide upon exposure to water in the gut.

WO2007/142542 discloses a treatment composition for treating bovine mastitis. The composition includes at least two components which have an isoelectric point above 6.8 and is extracted from milk or a milk derived substance.

However, none of these disclosures has resulted in an effective formulation and method of treatment to safely and effectively treat microbial infection in internal regions of the animal, such as the case with mastitis. The reasons for this lack of effectiveness have not been identified.

All references, including any patents or patent applications cited in this specification are hereby incorporated by reference. No admission is made that any reference constitutes prior art. The discussion of the references states what their authors assert, and the applicants reserve the right to challenge the accuracy and pertinency of the cited documents. It will be clearly understood that, although a number of prior art publications are referred to herein, this reference does not constitute an admission that any of these documents form part of the common general knowledge in the art, in New Zealand or in any other country.

It is acknowledged that the term 'comprise' may, under varying jurisdictions, be attributed with either an exclusive or an inclusive meaning. For the purpose of this specification, and unless otherwise noted, the term 'comprise' shall have an inclusive meaning - i.e. that it will be taken to mean an inclusion of not only the listed components it directly references, but also other non-specified components or elements. This rationale will also be used when the term 'comprised' or 'comprising' is used in relation to one or more steps in a method or process.

It is an object of the present invention to address the foregoing problems or at least to provide the public with a useful choice.

Further aspects and advantages of the present invention will become apparent from the ensuing description which is given by way of example only.

### DISCLOSURE OF INVENTION

According to the present invention there is provided a medicament for use in the treatment or prevention of mastitis in a mammary gland of an animal as set out in claim 1.

The medicament includes an extended acting oxygen generating system. According to one embodiment of the invention the method is characterised by the step of
a) applying the medicament containing the extended acting oxygen generating system to an area affected by the microbial infection or associated condition in the low oxygen environment.

The inventors have determined many traditional treatments for microbial infection do not work well for mastitis. The reason they believe this occurs is that mastitis occurs in a low oxygen environment which is not the case for open exterior wounds and the like.

The inventors' studies have revealed that the Lp system is significantly enhanced in vitro in a more aerobic environment opposed to a more anaerobic environment. Given the limited source of oxygen within the mammary gland, an extending acting oxygen generating system may significantly improve the bacteriostatic/bactericidal effect of a formulation for mastitic treatment and other infection sites in the body that have low levels of oxygen as a result of infection.

The underlying inventive step of the current application is the clever use of an extended acting oxygen generating system. This system may also make use of the endogenous Lp (and substrates) provided within the animal's body. It is evident that an animal (e.g. cow) produces sufficient amounts of Lp and often many of the substrates needed to make endogenous anti-microbial compounds. Eliminating the requirement of Lp and many of the substrates within the composition for treating mastitis serves a number of advantages such as:
1) cheaper and easier to manufacture the composition, and
2) it is more "natural" and therefore more appealing than antibiotics, and
3) It is less likely to cause a reaction/side effects within the mammary gland.

The inventors has identified that the invention may be utilised for substantially any microbial infection or associated condition which has a low oxygen content, not just mastitis. For example, the medicament may be used to treat infection at other parts on the body including an organ, glands, ducts, or any other area that falls prey to an microbial infection or an associated condition at a low oxygen environment. This could also be the case for small abcesses or pustules that would be present, for example, on the skin (e.g. pimples) but are encased or enclosed from atmospheric or systemic oxygen levels.

It is desirable to have available a product which is quick and easy to produce which effectively prevents or treats mastitis. What has been overlooked in the prior art is that, for the special case of mastitis, the 'engine' of the treatment formulation may already be present (Lp, Lf, leukocytes) naturally in milk and particularly in infected milk. However, a slow release oxygen source, to 'fuel' the action of the formulation, may be missing. This may be needed to produce effective amounts of endogenous peroxide (a subsequent substrate of the Lp reaction).

Throughout the specification the term "extended acting oxygen generating system" should include a substance or group of substances that can be used or combined to produce oxygen over a period longer than that occasioned by the normal exposure of the oxygen source to the environment into which the oxygen is to be released. For example, the oxygen generation may be a result of the medicament using a peroxide source, as discussed further below.

The inventors have discovered that it may be an extended acting oxygen generating system that is the limiting factor as an effective treatment for mastitis. Any supplementation of Lp or other milk defense proteins is not necessarily required, given that the infected milk already contains such components. In some instances, exogenous Lp substrates, such as thiocyanate, are included in the medicament.

Throughout the specification the term "low oxygen environment" should be taken as meaning any oxygen level which is lower that would be physiologically normal, due to infection, in any given location of an animal's body.

Throughout the specification the term "mammary gland" should be taken as meaning organs that, in mammals, produce milk.

In a preferred embodiment the mammary glands may be from goats (caprine), sheep (ovine), cows (bovine) or humans.

Throughout the specification the term "mastitis" should be taken as an inflammatory disease of the mammary gland of a mammal.

In a preferred embodiment, the medicament should be taken to include a mixture of two or more components, which include an extended acting oxygen generating system and an Lp substrate in the form of a halide or thiocyanate. The medicament may also include other components, which may improve or help ameliorate the effects of mastitis. In a preferred embodiment the medicament may also include other components, which may improve its pharmacokinetic properties.

Throughout the specification the term "treatment" should be taken as meaning "a medicament which prevents or ameliorates either a microbial infection or the effects or symptoms of a microbial infection, including any associated conditions."

Throughout the specification the term Lp system (or reaction) should be taken as meaning a peroxidase system which is able to use a peroxide source to form a antimicrobial product. Specific mention is provided in this specification in relation to the peroxidise system that utilises Lactoperoxidase (Lp), an enzyme present in mammary glands and milk (the location of mastitis)..

### OXYGEN PREFERABLY SUPPLIED BY A PEROXIDE SYSTEM

The extended acting oxygen generating system results from the inclusion of an extended peroxide generating system in the medicament.

The inventors have identified that some of the peroxide produced by this system may be able to break down to release oxygen to the infection site when the medicament is in use. This may help to increase the oxygen levels beyond what is normally present at the infection site (for example in the mammary gland as a result of mastitis).

The extended acting peroxide generating system is able to produce hydrogen peroxide.

In a particularly preferred embodiment, the extending acting peroxide generating system includes a component selected from the group consisting of percarbonate or urea peroxide (carbamide) and combinations thereof. Other examples of an extended acting peroxide generating system may include, but are not limited to, peroxide donors such as magnesium peroxide and calcium peroxide.

These peroxide generating systems do not require oxygen to form peroxide. As such, additional oxygen is unlikely to be needed to provide an effective bactericidal effect. For example, if the peroxide source is percarbonate or urea peroxide (carbamide), as discussed above, the applicants have found that exogenous oxygen may not be a requirement to provide beneficial anti-microbial results. Instead, the peroxide formed from this type of system may be sufficient to drive the Lp system without exogenous or endogenous oxygen present. However, provision of such a peroxide source may also increase levels of oxygen as a result of peroxide breakdown. A result of this may be boosting of the animal's natural peroxide generating system and therefore driving of the Lp reaction. (During infection, low oxygen levels normally would inhibit the Lp reaction).

Therefore, provision of an extending acting peroxide generating system may provide a synergistic effect. It not only drives the Lp reaction directly (peroxide is an Lp substrate), but is also may help to "jumpstart" the animal's natural defense system by increasing oxygen levels.

Other peroxide systems that do not require the need for oxygen to form peroxide may act in a similar fashion to those exemplified above and are thus considered within the scope of the invention.

### PREFERRED RATE OF OXYGEN AND/OR PEROXIDE RELEASE

The exact threshold levels of what defines "low" and "high" concentrations and "acceptable" or "unacceptable" release rates of either oxygen or peroxide may, of course, depend on individual mammary gland physiology, the exact state of infection, the time lapse since last milking and the volume of milk production within the gland, which would define a dilution rate when the medicament is injected. The rate of release, for example as described below, may be sufficient to achieve a resulting antimicrobial level of hypothiocyanite of approximately 100 micromolar, a level considered to be effective.

There is evidence to suggest that provision of ozone or superoxide can be used as an oxygen source to increase production levels of peroxide to treat infections.

The problem associated with such treatment is the oxygen release from such sources can be very quick (i.e. substantially instantaneous) - thus any peroxide produced as a result of added oxygen (as a substrate to the peroxide generating reaction) would likely be very limited, thereby hindering the subsequent Lp reaction.

Oxygen and peroxide are typically required at a low concentration as substrates for the Lp system. High concentrations of substrate may be inhibitory. Also oxygen has limited solubility in milk and if added in excess might be lost into the tissues.

The body's natural peroxide sources (e.g. NADH oxidase) are meant to be able to release peroxide in an extended fashion. However, in an oxygen depleted environment, such a system may not efficiently produce peroxide.

Some might argue that providing peroxide could solve the problem. However, too high levels of peroxide actually inhibit the Lp reaction which ultimately is needed to provide the antimicrobial agent.

Furthermore, peroxide in high concentration may be damaging to mammary tissues.

Therefore, one of the difficulties to address is providing oxygen (preferably using a peroxide generating source) release over an extended period of time and at appropriate levels in an oxygen depleted environment.

In a particularly preferred embodiment, the oxygen source is able to provide a slow release of oxygen.

In another particularly preferred embodiment, the extended acting peroxide generating system is able to provide a slow release of peroxide.

The inventors acknowledge that the Lp system requires peroxide between approximately 0.1 mM and 3 mM for optimal activity. With this in mind, the following ranges are provided as preferred embodiments.

Preferably, the rate of release is approximately 0.02 mg to 10 mg of peroxide per minute.

More preferably, the rate of release is approximately 0.1 mg to 0.5 mg of peroxide per minute.

The rate of release may be approximately 1 to 5 mg of peroxide over 30 - 60 minutes, which is considered to be a particularly preferred duration of the peroxide/oxygen release.

However, the actual duration of the peroxide release may depend on the condition to be treated.

In some circumstances, the duration may be quite short (although still longer than that occasioned by the normal exposure of the oxygen source to the environment into which the oxygen is to be released). For example, the minimal release duration is considered to be approximately 30 seconds. Such short bursts of peroxide release may be beneficial in treatment of mild cases of infection, such as pre-mastitis. Similarly, such a treatment could be used as a preventative treatment, even though the animal may not have any infection.

As an alternative example, for the treatment of severe mastitis, the rate of release may be extended to 14 hours to coincide with the time interval between milkings.

The inventors have identified that there are numerous methods known in the art to providing slow release of a compound. These include for example, encapsulation of a compound, granulation, coacervation, boluses, etc.

Preferably, the extending acting peroxide generating system includes a compound that is provided as granulated particles.

The inventors have identified that a particle size of approximately 425 - 250 µm is particularly beneficial in providing the preferred release rate of peroxide (0.02 - 10 mg H₂O₂ per minute) as well as providing a particle that can be delivered easily through a syringe.

The particles may be coated in a protective sheet such as cellulose, protein, biodegradable polymers, maltodextrin, pectin, polysaccharide, polyanions, polycations or gelatins. Alternatively, other compounds could be used, such as waxes, glycerides, monoglycerides, fatty acids, fatty alcohols, fatty esters, starch, alginate, mineral oils, vegetable oils or milk fats or proteins.

The compounds may also be stabilized with substances such as glycerol, sorbitol, mannitol, inositol, various buffers, acids, chelators, phosphates or stannates.

The inventors have identified that such a slow release compound could provide an appropriate peroxide release rate (preferably 1 - 5 mg over a 30 - 60 minute period). Although, adjusting the manufacturing parameters may allow the rate of release to be altered as appropriate.

### PREFERRED EXAMPLE OF AN EXTENDING ACTING OXYGEN / PEROXIDE GENERATING SYSTEM

The inventors now describe a particularly preferred example of an extending acting oxygen / peroxide generating system. However it should be acknowledged that the features and methods as described below may be used in a similar fashion to provide substantially any type of extending acting peroxide generating system.

Preferably, the extending oxygen / peroxide generating system may provide the following advantages:
- good release of peroxide (e.g. 1 - 3 milligrams per 30 - 60 minutes;
- is effective at a pH of approximately 6 - 7;
- the peroxide generating source does not overly effect the pH of the surrounding solution;
- the coated particles are stable on storage, particularly in a temperature of 40C;
- the preferred compositions are compatible with milk;
- studies indicate that the peroxide is able to increase oxygen levels, which is advantageous for treating microbial infection such as mastitis;
- effectively increases peroxide and / or oxygen levels at the site of administration over a sustained period of time.

### FURTHER PREFERRED EMBODIMENTS OF THE MEDICAMENT

To supplement the effectiveness of the formulation including the extending acting oxygen generating source, additional enzymes, substrates or other components may be added to the formulation, as discussed below.

In one embodiment, the medicament includes a lactoperoxidase (Lp) or functional equivalent thereof.

Throughout the specification the term "substrate" should be taken as meaning a molecule upon which an enzyme acts.

In one embodiment the medicament includes a substrate of an Lp enzyme from goats, sheep, cows, or humans acts upon.

In a preferred embodiment the medicament includes thiocyanate (SCN-), iodate or potassium iodide (KI).

The substrate is selected from the group consisting of thiocyanate or halide such as iodides or chlorides. The inventors have surprisingly identified that use of the Kl may be particularly advantageous over the use of other types of Lp substrates. This is surprising as Kl is not the most common substrate in the Lp reaction.

In one embodiment, the medicament includes glucose (GI) and glucose oxidase (GOD).

Gl and GOD require oxygen as a substrate to produce peroxide. However, as discussed above, in an infected mammary gland environment, oxygen supply may become more limited than in an uninfected mammary gland. In milk from healthy cows, for example, the oxygen concentration is typically about 1-2 ppm whereas the oxygen concentration may drop to 0.1 ppm in milk from infected quarters.

The medicament may utilize an endogenous source of oxygen. However, it is also possible that the oxygen used by GOD and glucose is actually derived from the extending acting oxygen generating system.
In a preferred embodiment the medicament includes exogenous milk-derived defense proteins.

Preferably, the medicament includes components derived from a "cationic fraction" purified from a milk source. Such a cationic fraction is known in the art, and is described in detail in granted New Zealand Patent No. 574229, which is hereby referred to entirely.

More specifically, the "cationic fraction" purified from a milk source should be taken to include at least two components which have an isoelectric point of or above substantially 6.8 and is extracted from milk, or a milk derived substance. In a preferred embodiment the cationic fraction includes at least lactoferrin, lactoperoxidase and angiogenin.

In another preferred embodiment, the cationic fraction includes chitinase-like protein -1 (CLP-1).

CLP-1 is similar in action to lysozyme, in that it lyses bacterial cell walls. Lactoferrin is immunomodulatory and has been shown to have some inhibitory effects. As a whole, the cationic fraction has good levels anti-inflammatory, antimicrobial and antioxidant activities. However, it is lacking its key substrates, oxygen and/or peroxide. In order to convey the bioactivity of the cationic fraction, one or both of these key substrates must be present.

In another preferred embodiment, biological salts may also be added to retain the isotonicity with milk.

The medicament may also include other compounds which may include antibiotics and analgesics.

The medicament may include at least one or more of the following: carriers, buffers, preservatives, excipients or other pharmaceutically acceptable components required to ensure the extended acting oxygen generating system is in a form that is easily dispensed, used and is efficient for the purpose of preventing or treating mastitis.

In one embodiment the medicament may also include at least one component which is capable of controlling the time release of the composition. This may be used to effectively treat mastitis over an extended period of time. Known components which could be used for this purpose would be well known to one skilled in the art.

In a preferred embodiment the extended acting oxygen generating system may be mixed with an inert liquid carrier.

In the embodiment where the final treatment composition is for use as a teat seal the final composition may also incorporate any 'hardening' component added to block the teat canal and physically prevent microbes from entering same. In some instances the final treatment composition configured for use as a teat seal may become substantially more solid when placed in the teat canal, thereby also physically preventing the entry of microorganisms.

Some or all of the components of the medicament are kept separated prior to administration. This may help avoid loss of the beneficial effects of the medicament before it is delivered to the site of infection. For example, combination of the GI/GOD prior to delivery (during storage) may reduce the potential availability of peroxide within the composition after delivery.

A delivery system for the composition separates the components in the medicament. Upon delivery, the components may come into direct contact, and allow production over time of oxygen/peroxide required for the Lp reaction in the mammary gland.

### PREFFERED EMBODIMENTS - METHOD OF TREATMENT

The typical method for applying a medicament for the treatment or prevention of mastitis is to apply the treatment compound into at least one mammary gland. If the animal is a dairy cow, the treatment compound may be applied into at least one bovine teat. The extended acting oxygen generating system may be used to form part of a final treatment composition. However this should not be seen as limiting as in some instances the extended acting oxygen generating system alone may be administered for the prevention and treatment of bovine mastitis.

For use in a milking cow the medicament may be resuspended with an aqueous solution, such as a buffer. This more dilute form could be prepared from a concentrate, which was made from a concentration step, for example, by ultrafiltration.

In one preferred embodiment the treatment composition may be in the form of granulated particles which are re-suspended in a solution prior to delivery to the infection site via injection.

In an alternative embodiment, the treatment composition is kept as a two-part formulation with a liquid part containing the substrates, salts, cationic fraction or any part thereof and a dry part containing the extended acting oxygen system, so that the latter remains dry until infused.

This may include for example: teat sprays, teat wipes, udder/teat washes, milking cluster backflush solutions or intramammary formulations for either lactating or non-lactating animals.

Liquid treatments for use in a milking cow may be massaged or applied up into the udder after milking.

However this should not be seen as limiting, as the final treatment composition could also be in the form of an oil, an emulsion, a powder, a gel or cream or as a solid putty like material.

In an alternative embodiment the final composition may be in the form of a teat seal. One skilled in the art would realize that the teat seal formulation may be in a range of configurations, for example, it may solidify after application, or may in a more solid form. The teat seal type of treatment is typically applied near or within the teat canal entrance.

Currently, it is anticipated that the dosage regime of the composition of the present invention may be within the range of 10 ml after each or every other milking for one to three days.

Throughout this specification the term final treatment composition should be taken as meaning the form in which the extended acting oxygen generating system is administered to the animal.

In a preferred embodiment the extended acting oxygen generating system of the present invention may be used to treat cows during the drying off or dry period.

In an alternative embodiment the extended acting oxygen generating system may be utilized during the milking or lactation period.

Aspects of the present invention have been described by way of example only and it should be appreciated that modifications and additions may be made thereto without departing from the scope thereof.

### BRIEF DESCRIPTION OF DRAWINGS

Further aspects of the present invention will become apparent from the following description which is given by way of example only and with reference to the accompanying drawings in which:
Figure 1 The effect of addition of hydrogen peroxide on the growth of Staphylococcus aureus;

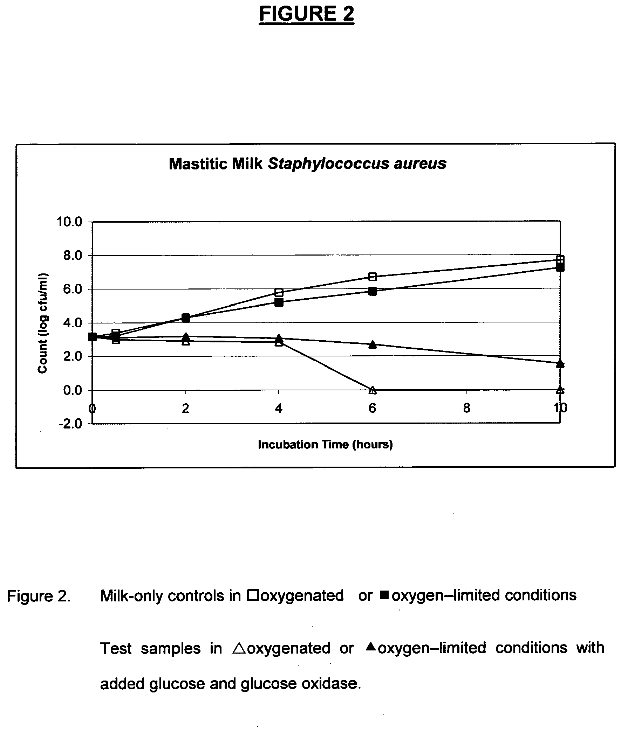

Figure 2 The effect of glucose and glucose oxidase on the growth of Staphylococcus aureus; and

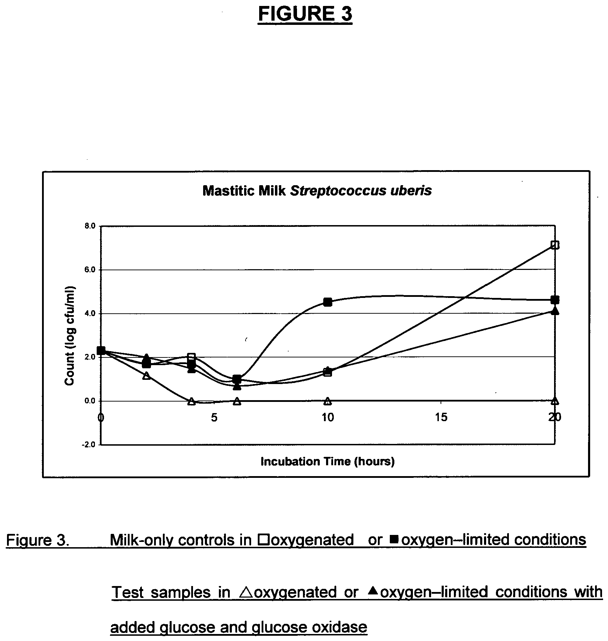

Figure 3 The effect of glucose and glucose oxidase on the growth of Streptococcus uberis.
Figure 4 The pH, oxygen and peroxide analysis over 200 minutes after addition of the Extended Acting Peroxide Generating Source to milk
Figure 5 The effect of a preferred Extending Acting Peroxide Generating Source
Figure 6 The effect of two different Lp substrates

### BEST MODES FOR CARRYING OUT THE INVENTION

### Introduction

In an infected mammary gland there is a demand for oxygen from both the growing organisms and the neutrophils trying to control their growth. There is a limit to the rate of transfer of oxygen into the gland, and therefore in moderate and severe mastitis the oxygen concentration in milk is reduced.

### Methods

Milk was collected at the morning milking, using aseptic technique, from a quarter that had been identified as having a sub-clinical infection with a single organism. The milk was taken to the laboratory and within 2 hours of collection divided amongst sterile incubation containers. The container was either a sealed vessel that was filled so there was no head-space and could be sampled through a rubber septum (limited oxygen) or a flask with a large surface area and head space, incubated in a shaking incubator (oxygenated). Because milk is aerated during collection and handling the samples should have the same oxygen content at the start of incubation. The growth rate was determined by sampling at regular intervals, diluting into recovery diluent, plating and counting the number of colonies.

Milk was collected from animals infected with either Staphylococcus aureus or Streptococcus uberis by technicians skilled in sterile collection and identification of mastitis organisms.

### Example 1:

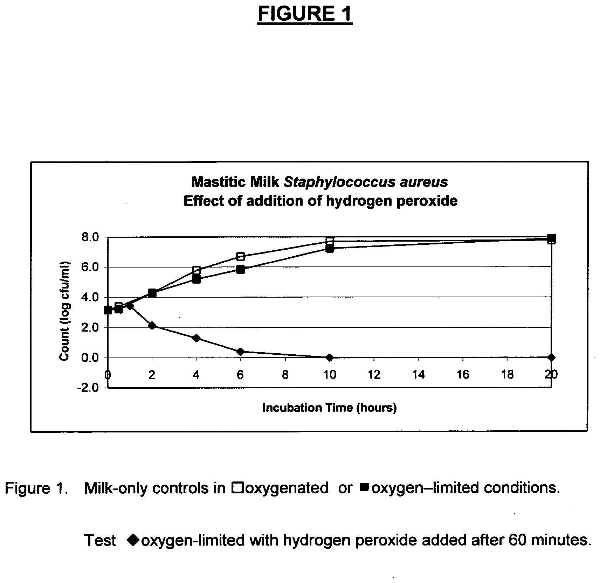

Figure 1 shows the effect of the direct addition of hydrogen peroxide on the growth of Staphylococcus aureus in mastitic milk. Both the oxygenated and oxygen-limited controls grew to around 10⁶ cfu per ml after 6 hours and to nearly 10⁸ cfu per ml after 20 hours. Hydrogen peroxide was added to the test sample at a final concentration of 10 micromoles per ml after the sample had been incubating for 60 minutes in oxygen-limited conditions. The number of viable organisms decreased from around 3000 cfu per ml milk at 60 minutes to 20 cfu per ml after 4 hours and no detectable organisms at 10 hours and thereafter.

This example shows that in the presence of limited oxygen, growth of Staphylococcus aureus is substantially inhibited by the addition of hydrogen peroxide and actual cell count decreases substantially to 0 after approximately 10 hours of the addition of hydrogen peroxide. This demonstrates that hydrogen peroxide may be useful basis for a treatment of microbial infection. It appears that the hydrogen peroxide lasted for a while as the bacterial numbers declined steadily for five hours after the addition of peroxide. However, in the mammary gland the peroxide would probably be destroyed as soon as it came in contact with the tissues and, therefore, would not last this long. Hence, there is a remaining need for an extended acting oxygen/peroxide generating system for in vivo application.

Example 2: Example 2 shows the effect of glucose and glucose oxidase (as a hydrogen peroxide generating system) on the growth of Staphylococcus aureus. Both the oxygenated and oxygen-limited controls grew to greater than 10⁷ cfu per ml after 10 hours. When glucose and glucose oxidase were included in oxygenated conditions there were no viable cells after 6 hours and thereafter, while there were still viable cells after 10 hours in oxygen-limited conditions. This shows that in the presence of a hydrogen peroxide generator (GL + GOD), growth of Staphylococcus aureus is significantly hindered in an oxygenated environment (i.e., using an exogenous 02 source) when compared to a sample with oxygen-limited conditions.

### Example 3

This example shows the effect of glucose and glucose oxidase (as a source of hydrogen peroxide) on the growth of Streptococcus uberis. After a growth lag of six hours the oxygen-limited control grew to greater than 10⁷ cfu per ml after 20 hours, while the oxygenated control increased to only 4 x 10⁴ cfu per ml. This may be an indication that this organism has a preference for microaerophilic conditions. When glucose and glucose oxidase were included in oxygenated conditions there were no viable cells after 4 hours and thereafter, while in oxygen-limited conditions growth increased to greater than 10⁴ cfu per ml after 20 hours.

### Introduction to Examples 4 - 11

An appropriate system was developed for delivering hydrogen peroxide and/or oxygen to an infected site such as the udders of cows, based on the inventive concept of the present invention.

The aim was to either supplement the oxygen level in milk to a level at or above the natural endogenous concentration, and to do this over an extended time period due to continuous consumption in the gland, Mayer et al. report that the oxygen concentration is 32.8 microM (1.03 µg/ml) in normal milk and 1.9 µM (0.06 µg/ml) in mastitic milk. The Lp system requires peroxide between 0.1 and 3mM for optimal activity.

Examples 4-7 outline the support data provided by the UniSA describing a particularly preferred peroxide generating source to be used for the present invention.

This source appears to release peroxide at an appropriate rate, is functional in the preferred pH range of 6 - 7, and is compatible with milk (which is advantageous particularly in the preferred treatment of mastitis).

### Example 4: Evaluation of Coated Sodium Percarbonate Particles

The aim of the peroxide generating source is to deliver 30 mg of nascent oxygen or peroxide into the growing volume of milk present in the udder, so that peroxide may be released slowly over a period of up to 14 hours.

A volume of 10 ml_ can be injected into the mammary gland following milking. The residual milk in the gland is roughly 200 ml, increasing up to 2 L.

It was found that approximately 70 -100 mg of sodium percarbonate is required to deliver 30 mg peroxide.

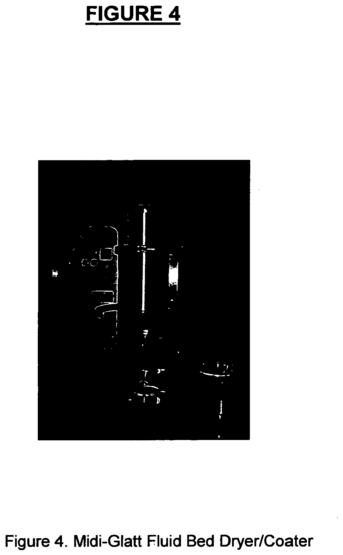

Sodium percarbonate coated with ethyl cellulose was added to 20 mL pasteurized and homogenized milk. The pH value (by pH Meter), the concentration of oxygen (by potentiometric electrode) and peroxide (by chemical test strips) were measured at interval sampling time, as shown in Figure 4A-C.

The pH level rose only marginally (from approximately 6.6 to 7.0) over the 200 minute testing period.

The oxygen concentration rose quickly from approximately 4.0 to 7.5 mg/L over 50 minutes, then levelled off at approximately 8.0 mg/L over the remaining 150 minutes of the testing period. This suggests that the peroxide source may be valuable in increasing oxygen levels to an appropriate level over an extended period of time (while not raising levels too high).

The peroxide concentration rose significantly from 0 ppm to 140 ppm over the 200 minute testing period. This suggested the peroxide source will be efficient at providing an extending acting peroxide generating source over an extended period of time (whilst not raising peroxide levels to extreme levels).

### Example 5: Storage Stability

The suggested storage temperature of the coated sodium percarbonate is lower than 40C to avoid slow decomposition of sodium percarbonate.

The particles are unlikely to aggregate when stored under dry conditions. It is important to keep the compound dry to prevent premature release.

### Example 6: Effect of Peroxide Generating Source

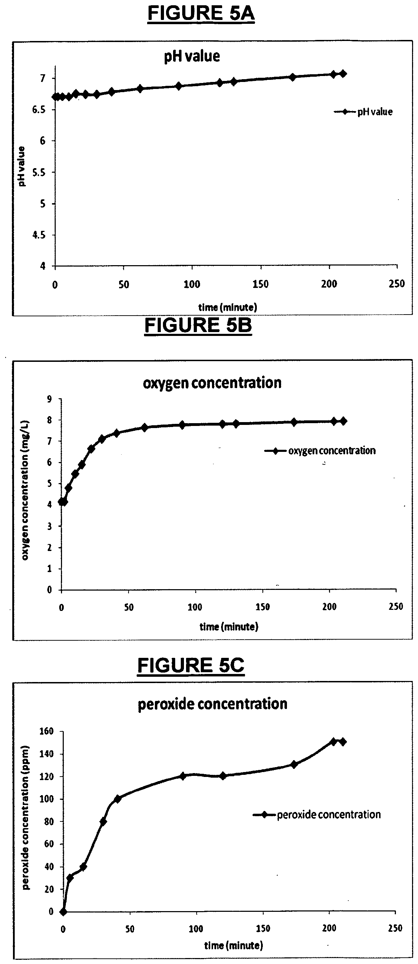

This study shows bacterial growth (recorded as log10 colony forming units/ml) in milk collected in a sterile manner from a lactating cow with sub-clinical Streptococcus uberis mastitis. The milk was divided between sealed sterile containers (oxygen limited environment). The rate of growth in an untreated control was compared with samples treated with a base formulation with and without a slow releasing peroxide generating source compound (results shown in Figure 5).

### Example 7: Comparison of Lp Substrates

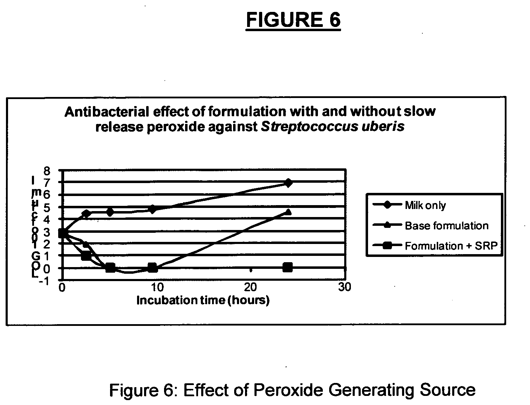

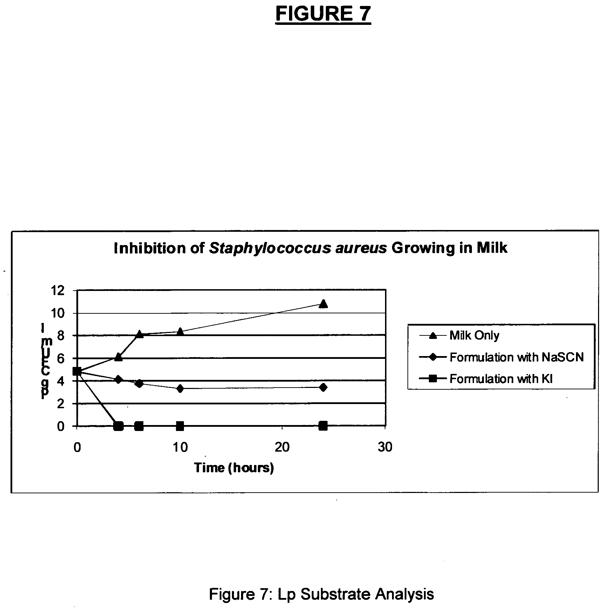

Figure 6 shows bacterial growth (recorded as log10 colony forming units/ml) in milk collected in a sterile manner from a lactating cow with sub-clinical Staphyloccus aureus mastitis. The milk was divided between sealed sterile containers (oxygen limited environment). The rate of growth in an untreated control was compared with samples treated with the formulation containing cationic fraction, slow release peroxide and either sodium thiocyanate (NaSCN) or potassium iodide (KI).

Aspects of the present invention have been described by way of example only and it should be appreciated that modifications and additions may be made thereto without departing from the scope thereof.

## Claims

1. A medicament for use in the treatment or prevention of mastitis wherein the oxygen level in the mammary gland is lower than that would be physiologically normal due to infection;
wherein the medicament includes an extended acting oxygen generating system
wherein the extended acting oxygen generating system includes a slow release hydrogen peroxide generation system, and
wherein the medicament also includes a substrate for a lactoperoxidase, the substrate in the form of a halide or thiocyanate,
**characterised in that**
the extended acting oxygen generating system is configured to provide oxygen generation over a time period between 30 seconds to 14 hours following administration to the mammary gland in the animal, and
some or all of the components of the extended acting oxygen generating system are separated prior to administration in order to prevent oxygen generation until during or after administration.

2. The medicament as claimed in claim 1 wherein the slow release hydrogen peroxide generation system provides peroxide generation at a rate of 0.02 to 10 mg peroxide per minute.

3. The medicament as claimed in claim 1 or claim 2 wherein the slow release hydrogen peroxide generation system provides peroxide generation at a rate of 1 to 5 mg peroxide per 30 to 60 minutes.

4. The medicament as claimed in any one of claims 1 - 3 wherein the slow release hydrogen peroxide generation system peroxide at a concentration between 0.1 mM and 3 mM.

5. The medicament as claimed in any one of claims 1 - 4 wherein the slow release hydrogen peroxide generation system includes a compound selected from the group consisting of a percarbonate or a carbamide, including sodium percarbonate, urea peroxide, magnesium peroxide or calcium peroxide and combinations thereof.

6. The medicament as claimed in any one of the above claims wherein the medicament includes glucose oxidase and glucose.

7. The medicament as claimed in claim 1 wherein the halide is an iodide or chloride.

8. The medicament as claimed in any one of the above claims wherein the medicament includes at least one type of biological salt to help retain the isotonicity of the treatment site.

9. The medicament as claimed in any one of claims 1 to 8 wherein the medicament composition is kept as a two-part formulation with a liquid part and a dry part, wherein the latter part contains an extended acting oxygen system, so that the latter remains dry until administered to the mammary gland in the animal.

10. The medicament for use as claimed in any one of claims 1-9 wherein the medicament is provided in a dosage regime of approximately 10 ml after each or every other milking for one to three days.

## Patentansprüche

1. Medikament zur Verwendung bei der Behandlung oder Verhütung von Mastitis, bei der das Sauerstoffniveau in der Brustdrüse aufgrund einer Infektion niedriger ist als das, das physiologisch normal wäre;
wobei das Medikament ein länger wirkendes sauerstofferzeugendes System beinhaltet,
wobei das länger wirkende sauerstofferzeugende System ein Wasserstoffperoxid-Erzeugungssystem mit langsamer Freisetzung beinhaltet und
wobei das Medikament auch ein Substrat für eine Lactoperoxidase beinhaltet, wobei das Substrat in Form eines Halogenids oder Thiocyanats vorliegt,
**dadurch gekennzeichnet, dass**
das länger wirkende sauerstofferzeugende System so konfiguriert ist, dass es eine Sauerstofferzeugung über einen Zeitraum von 30 Sekunden bis 14 Stunden nach Verabreichung an die Brustdrüse eines Säugetiers erbringt, und
einige oder alle der Komponenten des länger wirkenden sauerstofferzeugenden Systems vor der Verabreichung getrennt sind, um eine Sauerstofferzeugung zu verhindern, so dass sie erst während oder nach der Verabreichung erfolgt.

2. Medikament nach Anspruch 1, wobei das Wasserstoffperoxid-Erzeugungssystem mit langsamer Freisetzung eine Peroxiderzeugung mit einer Geschwindigkeit von 0,02 bis 10 mg Peroxid pro Minute erbringt.

3. Medikament nach Anspruch 1 oder 2, wobei das Wasserstoffperoxid-Erzeugungssystem mit langsamer Freisetzung eine Peroxiderzeugung mit einer Geschwindigkeit von 1 bis 5 mg Peroxid pro 30 bis 60 Minuten erbringt.

4. Medikament nach einem der Ansprüche 1 bis 3, wobei das Wasserstoffperoxid-Erzeugungssystem mit langsamer Freisetzung eine Peroxid in einer Konzentration zwischen 0,1 mM und 3 mM [sie].

5. Medikament nach einem der Ansprüche 1 bis 4, wobei das Wasserstoffperoxid-Erzeugungssystem mit langsamer Freisetzung eine Verbindung beinhaltet, die ausgewählt ist aus der Gruppe bestehend aus einem Percarbonat oder einem Carbamid, inkl. Natriumpercarbonat, Harnstoffperoxid, Magnesiumperoxid oder Calciumperoxid und Kombinationen davon.

6. Medikament nach einem der obigen Ansprüche, wobei das Medikament GlucoseOxidase und Glucose beinhaltet.

7. Medikament nach Anspruch 1, wobei das Halogenid ein Iodid oder Chlorid ist.

8. Medikament nach einem der obigen Ansprüche, wobei das Medikament wenigstens einen Typ von biologischem Salz beinhaltet, um die Beibehaltung der Isotonizität der Behandlungsstelle zu unterstützen.

9. Medikament nach einem der Ansprüche 1 bis 8, wobei die Medikamentenzusammensetzung als zweiteilige Formulierung mit einem flüssigen Teil und einem trockenen Teil aufbewahrt wird, wobei der letztere Teil ein länger wirkendes Sauerstoffsystem enthält, so dass Letzterer bis zur Verabreichung an die Brustdrüse des Säugetiers trocken bleibt.

10. Medikament zur Verwendung nach einem der Ansprüche 1 bis 9, wobei das Medikament im Rahmen eines Dosierungsregimes von etwa 10 ml nach jedem oder jedem zweiten Melken für ein bis drei Tage bereitgestellt wird.

## Revendications

1. Médicament pour utilisation dans le traitement ou la prévention de la mastite dans lequel le taux d'oxygène dans la glande mammaire est plus bas que celui qui aurait été physiologiquement normal en cas d'infection ;
le médicament contenant un système de génération de l'oxygène à action prolongée
ledit système de génération de l'oxygène à action prolongée contenant un système de génération du peroxyde d'hydrogène à libération lente, et
le médicament contenant également un substrat pour une lactopéroxidase, le substrat se présentant sous la forme d'un halogénure ou d'un thiocyanate,
**caractérisé en ce que**
le système de génération de l'oxygène à action prolongée est configuré pour fournir une génération d'oxygène pendant une période de temps de 30 secondes à 14 heures suite à une administration à la glande mammaire de l'animal, et
certains des composants du système de génération de l'oxygène à action prolongée sont séparés avant l'administration de manière à empêcher la génération d'oxygène jusqu'à pendant ou après l'administration.

2. Médicament tel que revendiqué dans la revendication 1 dans lequel le système de génération du peroxyde d'hydrogène à libération lente fournit une génération de peroxyde à un débit de 0,02 à 10 mg de peroxyde par minute.

3. Médicament tel que revendiqué dans la revendication 1 ou la revendication 2, dans lequel le système de génération du peroxyde d'hydrogène à libération lente fournit une génération de peroxyde à un débit de 1 à 5 mg de peroxyde toutes les 30 à 60 minutes.

4. Médicament tel que revendiqué dans l'une quelconque des revendications 1 à 3 dans lequel le système de génération du peroxyde d'hydrogène à libération lente fournit du peroxyde à une concentration comprise entre 0,1 mN et 3 mM.

5. Médicament tel que revendiqué dans l'une quelconque des revendications 1 à 4 dans lequel le système de génération du peroxyde d'hydrogène à libération lente contient un composé sélectionné dans le groupe constitué d'un percarbonate ou d'un carbamide, contenant du percarbonate de sodium, du peroxyde d'urée, du peroxyde de magnésium ou du peroxyde de calcium et des combinaisons de ceux-ci.

6. Médicament tel que revendiqué dans l'une quelconque des revendications précédentes dans lequel le médicament contient de l'oxydase de glucose et du glucose.

7. Médicament tel que revendiqué dans la revendication 1 dans lequel l'halogénure est un iodure ou du chlorure.

8. Médicament tel que revendiqué dans l'une quelconque des revendications précédentes dans lequel le médicament contient au moins un type de sel biologique pour aider à conserver l'isotonicité du site de traitement.

9. Médicament tel que revendiqué dans l'une quelconque des revendications 1 à 8 dans lequel la composition du médicament est maintenue sous forme de formulation en deux parties avec une partie liquide et une partie sèche, la dernière partie contenant un système de génération de l'oxygène à action prolongée, de sorte à ce que la dernière partie puisse rester sèche jusqu'à ce qu'elle soit administrée à la glande mammaire de l'animal.

10. Médicament pour utilisation selon les revendications de l'une quelconque des revendications 1 à 9, le médicament étant fourni à une posologie d'environ 10 ml après chaque traite ou après une traite sur deux pendant un à trois jours.
